# NEUE EUROPÄISCHE PATENTSCHRIFT

(11) **EP 1 438 360 B2**
(45) Veröffentlichungstag und Bekanntmachung des Hinweises auf die Entscheidung über den Einspruch: **23.01.2013**
(45) Hinweis auf die Patenterteilung: 09.04.2008
(21) Anmeldenummer: 02779430.4
(22) Anmeldetag: 27.09.2002
(51) Int. Cl.: C09C 1/00

(54) **PIGMENT MIT METALLGLANZ**
PIGMENT WITH A METALLIC LUSTRE
PIGMENT A ECLAT METALLIQUE

(30) Priorität: 27.10.2001 DE 10153196
(43) Veröffentlichungstag der Anmeldung: 21.07.2004
(73) Patentinhaber: Merck Patent GmbH, 64293 Darmstadt (DE)
(72) Erfinder: PFAFF, Gerhard, 64389 Münster (DE); ANDES, Stephanie, 63452 Hanau (DE); UHLIG, Michael, 64297 Darmstadt (DE); FRIZ, Martin, 64297 Darmstadt (DE); VOGT, Reiner, 64289 Darmstadt (DE); NITTA Katsuhisa, Iwaki-shi 971-8152 (JP)
(86) Internationale Anmeldenummer: PCT/EP2002/010864
(87) Internationale Veröffentlichungsnummer: WO 2003/037993

(56) Entgegenhaltungen:
- EP-A- 1 045 014
- EP-A1- 0 761 600
- EP-A1- 1 013 722
- EP-A1- 1 013 724
- EP-A1- 1 013 725
- EP-A2- 0 763 573
- EP-A2- 1 148 028
- WO-A-01/40383
- WO-A-93/08237
- JP-A- 04 039 362
- JP-A- 10 259 317
- JP-A- 54 073 341
- JP-A- 63 043 962
- US-A- 3 536 520
- US-A- 5 436 077
- US-A- 5 702 519
- US-B1- 6 267 810
- R.GLAUSCH ET AL: 'Special Effect Pigments', 1998, CURT R.VINCENTZ VERLAG, HANNOVER Seiten 30-31 52 - 54
- G.PFAFF ET AL: 'New effect pigments using innovative substrates' EUR.COAT.J. April 1999, Seiten 90 - 96
- G.PFAFF ET AL: 'Welt der Farben', 2000 Seiten 16 - 19

## Beschreibung

Die Erfindung betrifft ein Effektpigment, das aus einem plättchenförmigen, Substrat aus Aluminiumoxid und einer Metallschicht besteht, die das Substrat vollständig umhüllt. Gegenstand der Erfindung ist weiterhin ein Verfahren zur Herstellung des Pigmentes sowie dessen Verwendung.

Metalleffektpigmente werden seit vielen Jahren in Beschichtungen zur Erzeugung des Metalleffektes eingesetzt. Klassische Metalleffektpigmente bestehen aus plättchenförmigen Metallpartikeln, deren optische Wirkung auf der gerichteten Reflexion von einfallendem Licht an den idealer Weise flächig ausgebildeten und im jeweiligen Anwendungsmedium parallel ausgerichteten Metallteilchen beruht.

Hauptanwendungsgebiete der Metalleffektpigmente sind die Automobil-und die Druckindustrie. Daneben werden sie auch für die Einfärbung von Kunststoffen und Anstrichmitteln, für Lederbeschichtungen, in der Kosmetik und in der Keramikindustrie eingesetzt. Im Autolackbereich werden sie vor allem für die Erzeugung des Metallic-Effektes verwendet, wobei sie zumeist gemeinsam mit anderen Pigmenten wie Perlglanzpigmenten, Titandioxid, Ruß oder organischen Pigmenten im Lack appliziert werden.

Um die Güte von Metallic- bzw. Metall-Effekten beispielsweise in Lacken, Druckfarben und Kunststoffen miteinander vergleichen zu können, wurden bestimmte Qualitätsmerkmale festgelegt. Diese sind unter anderem die Brillanz (Sparkle-Effekt und metallischer Glanz), die Helligkeit sowie der Flop (Helligkeitsänderung in Abhängigkeit vom Betrachtungswinkel), die Abbildeschärfe, die Bunttonsättigung bei farbigen metallischen Beschichtungen und das Deckvermögen. Der Metalleffekt wird wesentlich von der Teilchenform und dem Formfaktor (Verhältnis mittlerer Teilchendurchmesser zu mittlerer Teilchendicke) der Pigmente sowie von ihrer Oberflächenglätte, der Partikelgröße, der Teilchengrößenverteilung und von der Pigmentorientierung parallel zur Oberfläche des Lackes oder Kunststoffes und dergleichen beeinflusst.

Der optische Eindruck bestimmt sich nach dem Verhältnis von reflektiertem zu gestreutem Licht. Während bei größeren Pigmentteilchen mit gleichmäßiger Form eine stärkere Reflexion auftritt, die sich in einer hohen metallischen Brillanz, einer verbesserten Helligkeit und starkem Flop äußert, ist der Streuanteil bei feinen Pigmenten mit unregelmäßiger Teilchenstruktur sehr hoch, was zu einer verbesserten Abbildeschärfe und einer guten Deckfähigkeit führt.

Es besteht nun aber ein Bedarf an Metallpigmenten, die gleichzeitig sowohl über eine hohe Brillanz als auch über eine gute Deckfähigkeit und Abbildeschärfe verfügen. Dies kann von den gegenwärtig verfügbaren Metalipigmenten nicht geleistet werden, da abhängig von der Teilchengrößenverteilung jeweils gegenläufige Effekte beobachtet werden.

So erfüllen die traditionell eingesetzten Aluminium-Pigmente, die durch mechanische Verfahren ausgehend von Aluminium-Grieß hergestellt werden, diese Anforderungen nicht. Je nach Ausgangsmaterial und Mahltechnik werden ungleichförmige Aluminiumplättchen mit einem hohen Streuanteil oder eher runde Aluminiumplättchen erhalten. Die runden Plättchen (sog. Silberdollars) besitzen eine relativ glatte Oberfläche und können wegen ihres geringen Streuanteils zur Erzielung verbesserter metallischer Glanz- und Glitzereffekte eingesetzt werden. Bei den vergleichsweise großen Partikeldurchmessern kann jedoch kein gutes Deckvermögen erzielt werden. Silberdollars werden zum Beispiel unter der Bezeichnung Stapa® Metallux 2000 von der Firma Eckart angeboten.

Es wurden jedoch auch besonders dünne Aluminiumplättchen entwickelt, welche über ein PVD-Verfahren ( Physical Vapour Deposition) hergestellt werden. Diese werden in US 3 949 139 und US 4 321 087 näher beschrieben. Während übliche Aluminiumpigmente Plättchendicken über 100 nm besitzen, weisen PVD-Pigmente, beispielsweise die Type Metallure ® der Firma Eckart, Dicken von deutlich unter 100 nm auf. Mit den PVD-Aluminiumpigmenten ist im Vergleich zu den herkömmlichen Aluminiumpigmenten ein verbessertes Deckvermögen sowie ein Metallfolien-artiger Effekt erzielbar.

Ihre Anwendung erfordert jedoch ein sehr spezielles anwendungstechnisches Know-how, um reproduzierbare Effekte zu erzielen.
Andernfalls treten bei der Applikation der Pigmente Probleme auf. So können sie infolge ihrer geringen mechanischen Stabilität bei der Einarbeitung in Anwendungssysteme, beispielsweise Lacke oder Kunststoffe, nur geringen Scherkräften ausgesetzt werden. Zudem ist ihre Verwendung in wasserbasierenden Anwendungssystemen wegen der herstellungsbedingten großen reaktiven Oberfläche problematisch. Für bestimmte Anwendungen sind sie darüber hinaus überhaupt nicht geeignet, beispielsweise für die Pulverlackierung.

Es hat auch nicht an Versuchen gefehlt, Metalleffekte mittels Pigmenten zu erzielen, die keine Metallpigmente im herkömmlichen Sinne sind, zum Beispiel mit den klassischen Interferenzpigmenten, welche aus transparenten Substraten und darauf abgeschiedenen Metalloxidschichten bestehen oder mit Interferenzpigmenten, welche auf einem Metallkern eine oder mehrere Metall- und/ oder Metalloxidschichten aufweisen. Mit den ersteren kann meist ein guter, metallähnlicher Glanz erzeugt werden. Da die Substrate und alle darauf befindlichen Schichten jedoch zu einem hohen Anteil transparent sind, verfügen sie nur über ein schwaches Deckvermögen.

Die Pigmente aus einem Metallkern mit einer oder mehreren Metall- und/ oder Metalloxidschichten zeigen in der Regel ein gutes Deckvermögen und einen lebhaften Farbflop. Die Verfahren zur Herstellung solcher Pigmente sind jedoch außerordentlich aufwendig und kostenintensiv.

Aus EP 0 763 573 ist ein Perlglanzpigment bekannt, welches aus einem Substrat aus Aluminiumoxid mit einem Anteil an Titanoxid und einer darauf befindlichen Schicht aus einem Metalloxid besteht. Dieses Pigment ist transparent und weist einen hohen Perlglanz auf. Es wird von der Firma Merck KGaA unter dem Namen Xirallic ® vertrieben.

Aus EP 1 013 724 ist bekannt, dass Pigmente vom Typ Xirallic ®, die gegebenenfalls auch mit Metallen statt Metalloxiden beschichtet sein können, in bestimmten Anwendungsgebieten vorteilhaft in Kombination mit speziellen Effektpigmenten, darunter Metallpigmenten, eingesetzt werden können. Diese Pigmentkombination soll zu einem bemerkenswerten Tiefenglanz, Glitzereffekten und einem starken Farbflop führen, was jedoch von der Zusammensetzung des Gemisches abhängig ist. Ein gutes Deckvermögen kann in dieser Mischung nur dann erzielt werden, wenn deckende Pigmente zugegeben werden.

Aus der US 6,267,810 B1 sind Pigmentmischungen aus wenigstens zwei Komponenten bekannt, wobei Komponente A aus Al₂O₃-Pigmenten besteht, die mit ein oder mehreren Metallen, Metalloxiden und/oder Metallsulfiden beschichtet sind, und Komponente B aus Spezialeffekt-Pigmenten besteht.

WO 01/40383 A1 offenbart ein Edelmetall-haltiges Farbeffektmaterial mit einem plättchenförmigen Substrat, welches von einer gegenüber einfallendem Licht hoch reflektiven ersten Schicht aus verschiedenen Edelmetallen, einer zweiten, die erste Schicht einhüllenden Schicht, die in Abhängigkeit vom Einstrahlwinkel des Lichtes variable Passlängen des Lichtes erzeugt, und einer für eingestrahltes Licht selektiv transparenten dritten Schicht umhüllt ist.

In der EP 1 045 014 A2 sind Pigmentmischungen beschrieben, die aus mindestens zwei Komponenten bestehen, wobei Komponente A Mehrschichtpigmente auf Basis von Glimmer, SiO₂-Plättchen, Glasplättchen, Al₂O₃-Plättchen oder Polymerplättchen und Komponente B plättchenförmige, nadelförmige oder sphärische Farbmittel oder Füllstoffe sind.

Es sind bisher keine Pigmente bekannt, mit denen gleichzeitig sowohl ein metallischer optischer Eindruck mit starkem Glanz, der glitzernde Effekte einschließt, als auch ein gutes Deckvermögen erzielt werden kann.

Es ist deshalb die Aufgabe der Erfindung, ein Pigment mit Metallglanz bereitzustellen, welches in allen Anwendungssystemen, wie Lacken, Kunststoffen und Druckerzeugnissen problemlos einsetzbar ist und diesen ohne Zugabe weiterer Pigmente ein deckendes, funkelndes metallisches Aussehen verleiht,

Diese Aufgabe wird gemäß der Erfindung durch ein Pigment mit Metallglanz gemäß Anspruch 1 gelöst.

Weiterhin wird diese Aufgabe gelöst durch ein Verfahren zur Herstellung dieses Pigmentes, indem das Substrat in einem wässrigen und/oder Lösemittel enthaltenden Medium in Anwesenheit einer Metallverbindung suspendiert und nach Zugabe eines Reduktionsmittels die Metallschicht auf dem Substrat abgeschieden wird.

Diese Aufgabe wird ebenfalls gelöst durch ein Verfahren zur Herstellung dieses Pigmentes, indem das in einem Wirbelbett fluidisierte Substrat mit Metallen beschichtet wird, die durch Gasphasenzersetzung der entsprechenden flüchtigen Metallverbindungen erhalten werden.

Schließlich wird diese Aufgabe gelöst durch ein Verfahren zur Herstellung dieses Pigmentes, indem im Hochvakuum durch Sputtern oder thermisches Verdampfen die entsprechenden Metalle auf dem Substrat abgeschieden werden, wobei das Substrat während des Beschichtungsvorgangs gleichmäßig in Bewegung gehalten wird.

Gegenstand der Erfindung ist weiterhin die Verwendung der erfindungsgemäßen Pigmente in Farben, Lacken, Druckfarben, Kunststoffen, kosmetischen Formulierungen, keramischen Materialien, Gläsern, Papier, zur Lasermarkierung von Kunststoffen, in Sicherheitsanwendungen sowie in Trockenpräparaten und Pigmentpräparationen.

Als Substrat für die erfindungsgemäßen Pigmente werden Plättchen aus Aluminiumoxid eingesetzt, wobei diese zusätzlich geringe Anteile Titanoxid enthalten.

Diese Aluminiumoxidplättchen werden nach besonderen Verfahren hergestellt und weisen deshalb eine besonders glatte Oberfläche auf. Sie besitzen eine Dicke zwischen 300 nm und 500 nm. Diese Partikeldicke kann über die Reaktionsbedingungen der Herstellungsverfahren eingestellt werden. Ist die Partikeldicke kleiner als 250 nm, kann der gewünschte Glitzereffekt der Pigmente nicht mehr erzielt werden, weil die Teilchen im Anwendungsmedium nicht mehr als Einzelpartikel wahrgenommen werden. Wenn jedoch die Partikeldicke 1 µm übersteigt, kann eine glatte, hochreflektierende Oberfläche im Anwendungsmedium nicht mehr gewährleistet werden, da die Gefahr besteht, dass sich die Pigmente ungünstig ausrichten und damit Oberflächenrauhigkeiten auftreten.

Bevorzugt sind die Al₂O₃ -Substrate, deren Aufbau und Herstellung in EP 0 763 573 beschrieben ist. Diese enthalten zwischen 0,1 und 4 Gew.- % an Titanoxid und besitzen vorzugsweise einen mittleren Teilchendurchmesser von 5-60 µm, eine Dicke von kleiner als 1 µm und einen Formfaktor von größer als 20.
Sie werden nach einem Verfahren hergestellt, bei welchem aus einer wässrigen Lösung eines wasserlöslichen Aluminiumsalzes in Anwesenheit eines wasserlöslichen Titansalzes sowie einer wässrigen Lösung, die ein Alkalimetallsulfonat und Phosphorsäure oder ein Phosphat enthält, die entsprechenden Einkristalle durch Verdampfung abgeschieden und anschließend bei hohen Temperaturen getrocknet werden.

Besonders bevorzugt sind jedoch die in Eur.Coat.J., 04/99, S.90-96, beschriebenen Substrate. Derartige, auch Alumina-Flakes genannte Al₂O₃-Substratpartikel werden über ein Kristallwachstumsverfahren aus der Salzschmelze synthetisiert. Dazu wird hydratisiertes Aluminiumoxid, welches mit Titanoxid und Phosphat dotiert ist, mit Natriumsulfat in wässriger Suspension gemischt und anschließend getrocknet, wobei ein homogenes Pulver erhalten wird. Dieses Pulver wird in Tiegeln auf bis zu 1200°C erhitzt. Dabei entstehen durch einen Kristallwachstumsmechanismus die Alumina-Flakes in Form von Einkristallen. Nach dem Abkühlen werden die löslichen Bestandteile entfernt und die Al₂O₃-Plättchen durch Filtration isoliert. Die so entstehenden plättchenförmigen Teilchen besitzen eine außerordentlich glatte Oberfläche und weisen sehr regelmäßige Kristallformen auf. Sie neigen nicht zur Zwillingskristallbildung oder Agglomeration und lassen sich sehr gut dispergieren.

Der mittlere Durchmesser der Substratteilchen ist an sich nicht kritisch. Üblicherweise liegt er im Bereich von 1 bis 250 µm, vorzugsweise 2 bis 200 µm und insbesondere 5 bis 60 µm.

Der Formfaktor der Substratteilchen ist größer als 20, bevorzugt jedoch zwischen 50 und 200.

Für die das Substrat umhüllende Metallschicht sind Metalle geeignet, die ein besonders starkes Reflexionsvermögen besitzen. Bevorzugt werden Aluminium, Titan, Chrom, Nickel, Silber, Zink, Molybdän, Tantal, Wolfram, Palladium, Kupfer, Gold, Platin oder diese enthaltende Legierungen, beispielsweise Hastelloy verwendet. Besonders bevorzugt sind Aluminium und Silber.

Die Dicke der Metallschicht liegt zwischen 15 und 100 nm. Sie wird bevorzugt auf 20 bis 50 nm eingestellt.

Die Metallschicht kann nasschemisch nach bekannten Verfahren durch Reduktion anorganischer oder organischer Metallverbindungen in einer Suspension der Substratpartikel aufgebracht werden. Sie kann aber auch durch ein CVD-Verfahren (Chemical Vapour Deposition), beispielsweise die Gasphasenzersetzung von Metallcarbonylen oder durch ein PVD-Verfahren, beispielsweise durch Sputtern oder Aufdampfen von Metallen, abgeschieden werden.

Bei der nasschemischen Abscheidung der Metallschicht wird das Substrat in einem wässrigen und/oder Lösemittel enthaltenden Medium in Anwesenheit einer Metallverbindung suspendiert und nach Zugabe eines Reduktionsmittels das Metall auf dem Substrat abgeschieden. Die Metallverbindung kann eine anorganische Verbindung, zum Beispiel Silbernitrat oder eine metallorganische Verbindung, beispielsweise Nickelacetylacetonat sein. Das zu verwendende Lösungsmittel wird durch die Löslichkeit der metallorganischen Verbindung bestimmt.

Das in US 3 536 520 beschriebene Verfahren arbeitet mit Nickelchlorid in wässriger Phase, wobei das Substrat (Glimmer) einer Vorbehandlung mit Zinn- und Palladiumchlorid unterzogen wird. Als Reduktionsmittel wird Hypophosphit verwendet.

In EP 0 353 544 werden als Reduktionsmittel für die nasschemische Metallabscheidung reduzierende Verbindungen, wie Aldehyde (Formaldehyd, Acetaldehyd, Benzaldehyd), Ketone (Aceton), Carbonsäuren und deren Salze (Weinsäure, Ascorbinsäure), Reductone (Isoascorbinsäure, Triosereducton, Reduktinsäure) und reduzierende Zucker (Glucose) genannt. Aber auch reduzierende Alkohole (Allylalkohol), Polyole und Polyphenole, Sulfite, Hydrogensulfite, Dithionite, Hypophosphite, Hydrazin, Borstickstoffverbindungen, Metallhydride und komplexe Hydride von Aluminium und Bor können verwendet werden.

Weiterhin kann die Abscheidung der Metallschicht mit Hilfe eines CVD-Verfahrens erfolgen. Derartige Verfahren sind bekannt. Bevorzugt werden hierfür Wirbelschichtreaktoren eingesetzt. EP 0 741 170 beschreibt die Abscheidung von Aluminiumschichten durch Reduktion von Aluminiumalkylen mit Kohlenwasserstoffen in einem Inertgasstrom. Weiterhin können die Metallschichten durch Gasphasenzersetzung der entsprechenden Metallcarbonyle in einem beheizbaren Wirbelschichtreaktor, wie er in EP 045 851 beschrieben ist, abgeschieden werden. Nähere Angaben zu diesem Verfahren sind in WO 93/12182 enthalten.

Ein weiteres Verfahren zur Abscheidung dünner Metallschichten, das im vorliegenden Falle für das Aufbringen der Metallschicht auf dem Substrat anwendbar ist, ist das bekannte Verfahren zum Aufdampfen von Metallen im Hochvakuum. Es ist in Vakuum-Beschichtung, Bände 1-5; Herausgeber Frey, Kienel und Löbl, VDI-Verlag 1995 ausführlich beschrieben.

Zur Herstellung der erfindungsgemäßen Glanzpigmente ist es unbedingt notwendig, das Hochvakuumbedampfungsverfahren an das in Pulverform vorliegende Substrat anzupassen. Dazu ist es erforderlich, dass das Substrat während des Bedampfungsverfahrens im Vakuumkessel gleichmäßig in Bewegung gehalten wird, um eine homogene Beschichtung aller Partikeloberflächen zu gewährleisten. Dies gelingt zum Beispiel durch den Einsatz von rotierenden Behältern oder die Verwendung von Vibrationsvorrichtungen.

Beim Sputterverfahren wird zwischen dem Träger und dem Beschichtungsmaterial, das in Form von Platten (Target) vorliegt, eine Gasentladung (Plasma) gezündet. Das Beschichtungsmaterial wird durch energiereiche Ionen aus dem Plasma, z.B. Argonionen, beschossen und dadurch abgetragen bzw. zerstäubt. Die Atome oder Moleküle des zerstäubten Beschichtungsmaterials werden auf dem Träger niedergeschlagen und bilden die gewünschte dünne Schicht.

Für Sputterverfahren eignen sich besonders Metalle oder Legierungen. Diese können mit vergleichsweise hohen Geschwindigkeiten, insbesondere im sogenannten DC-Magnetron-Verfahren, zerstäubt werden.
Letzeres wird in der vorliegenden Erfindung für das Aufbringen der Metallschicht auf den Substratpartikeln besonders bevorzugt.

Das Sputterverfahren ist beschrieben in Vakuum-Beschichtung, Bände 1-5; Herausgeber Frey, Kienel und Löbl, VDI-Verlag 1995.

Werden Pigmente mit farbigen Metalleffekten gewünscht ist es möglich, auf der Metallschicht noch weitere Schichten aus farbigen oder farblosen Metalloxiden, Metallnitriden, Metallsulfiden und/oder Metallen aufzubringen. Diese Schichten sind transparent oder semitransparent und lassen mindestens 10 % des einfallenden Lichtes durch.
Es ist bevorzugt, dass sich dabei Schichten mit hoher Brechzahl und Schichten mit niedriger Brechzahl abwechseln oder dass eine Schicht aufgebracht ist, welche innerhalb der Schichtdicke einen Brechzahlgradienten aufweist. Die dabei auftretenden Interferenzerscheinungen bewirken zusätzlich ein intensives Farbspiel und/ oder einen starken Farbflop, so dass sich die Pigmente in vielen Anwendungsbereichen vorteilhaft einsetzen lassen.

Für den Einsatz in Außenanwendungen, insbesondere bei der Applikation in Fahrzeuglackierungen, können die erfindungsgemäßen Pigmente mit einer zusätzlichen wetterstabilisierenden Schutzschicht, der sogenannten Nachbeschichtung, versehen werden, die gleichzeitig eine optimale Adaption an das Bindemittelsystem bewirkt. Derartige Nachbeschichtungen sind beispielsweise in EP 0 268 918 und EP 0 632 109 beschrieben worden.

Mit den erfindungsgemäßen Pigmenten ist es erstmals möglich, nur mit einem einzigen Pigment gleichzeitig sowohl einen starken metallischen Glanz als auch ein gutes Deckvermögen zu erzielen, wobei die damit beschichteten, bedruckten oder eingefärbten Produkte auch ein starkes Funkeln aufweisen, was insbesondere für Automobillacke sehr erwünscht ist. Es ist nicht bekannt, worauf dieser Effekt, der auch als "metallischer Kristalleffekt" bezeichnet werden kann, genau beruht. Es wird jedoch vermutet, dass die besonders glatte Oberfläche und die regelmäßige Kristallform der eingesetzten Substrate, die auch bei der nachfolgenden metallischen Beschichtung erhalten bleibt, zu einer erhöhten Reflexion des eingestrahlten Lichtes führt, weshalb im Vergleich zum bekannten Stand der Technik kleinere Pigmentoberflächen ausreichen, um einen hohen metallischen Glanz und große Helligkeitswerte sowie einen guten Helligkeitsflop zu erzielen. Die Pigmente sind jedoch groß genug, um zumindest zum Teil visuell als Einzelpartikel wahrgenommen zu werden, was zu einem strahlend funkelnden optischen Eindruck führt.
Die insgesamt aber vergleichsweise kleinen Durchmesser der Pigmente und die eingestellte Dicke der Partikel führen dazu, dass ausreichend diffus streuende Kanten vorhanden sind, die zu einem hohen Streulichtanteil und damit zu einer deckenden Beschichtung führen.

Neben den beschriebenen optischen Effekten zeigen die erfindungsgemäßen Pigmente auch eine Reihe anderer Vorteile. So ist zum Beispiel ihre mechanische Stabilität insbesondere im Vergleich zu den über PVD-Verfahren hergestellten Pigmenten des Standes der Technik durch das stabilisierende nichtmetallische Substrat stark verbessert. Dies äußert sich zum Beispiel in einer geringen Biegsamkeit, die stabil glatte Oberflächen garantiert, ohne dass diese brüchig werden. Die mechanische Stabilität sorgt dafür, dass in den verschiedenen Anwendungssystemen auch bei mechanischer Belastung der Partikel durch Rühren, Mahlen oder dergleichen keine Zerstörung der Pigmente stattfindet. Auch in den bekannten Pumpleitungssystemen lassen sie sich problemlos einsetzen, da sie nur eine geringe Absetzneigung zeigen. Überraschenderweise können sich die erfindungsgemäßen Pigmente im Anwendungssystem auch gut orientieren, obwohl sie eher mittlere Schichtdicken aufweisen.
Außerdem sind die beschriebenen Verfahren zur Herstellung der erfindungsgemäßen Pigmente einfach und vergleichsweise kostengünstig durchführbar.

Die erfindungsgemäßen Pigmente sind mit einer Vielzahl von Farbsystemen kompatibel, vorzugsweise im Bereich der Lacke, Farben und Druckfarben. Sie sind ebenfalls für die Lasermarkierung von Papier und Kunststoffen sowie in keramischen Materialien und für Anwendungen im Agrarbereich einsetzbar. Aufgrund ihrer besonderen Effekte sind sie jedoch insbesondere für den Automobilbereich, die Druckindustrie sowie die dekorative Kosmetik geeignet. Sie können ebenfalls bei der Herstellung von Pigmentpräparationen und Trockenpräparaten eingesetzt werden, welche insbesondere in Druckfarben und Lacken Verwendung finden. Ein bevorzugtes Einsatzgebiet ist auch der Sicherheitsbereich mit verschiedenen Anwendungen, zum Beispiel in Geldscheinen, Kreditkarten, Visa, für Steuerbanderolen oder dergleichen.

Obwohl die erfindungsgemäßen Pigmente selbst über exzellente Eigenschaften verfügen, können sie selbstverständlich auch in Abmischung mit den verschiedensten handelsüblichen Pigmenten, beispielsweise organischen oder anorganischen Farbstoffen, herkömmlichen transparenten, bunten, schwarzen oder weißen Pigmenten wie zum Beispiel metalloxidbeschichteten Glimmerpigmenten, mit holographischen Pigmenten, LCPs (Liquid Crystal Polymers) oder herkömmlichen Metallpigmenten verwendet werden. Außerdem können sie in jedem Verhältnis mit handelsüblichen Pigmenten und Füllstoffen und/ oder Bindemitteln gemischt werden.

Die vollständige Offenbarung aller vorstehend genannten Patentanmeldungen, Patente und Veröffentlichungen ist durch Bezugnahme in dieser Anmeldung enthalten.

Die nachfolgenden Beispiele sollen die Erfindung näher erläutern, ohne sie jedoch zu beschränken.

### Beispiele

### Beispiel 1:

Für eine Beschichtung von Aluminiumoxidplättchen mit Silber werden folgende konzentrierte Reaktionslösungen hergestellt:
Aktivierungskonzentrat:
   11g Sn(II)chlorid werden in 9g konzentrierter Salzsäure gelöst und mit voll entsalztem Wasser (VE-Wasser) auf 100g aufgefüllt.
Reduktionskonzentrat:
   5g D-Glucose und 5g D-Fructose werden in 100ml VE-Wasser gelöst.
Silberlösung:
   4g Kaliumhydroxidplättchen werden in 10ml VE-Wasser gelöst und langsam mit 16ml konzentriertem Ammoniak versetzt. 4g Silbernitrat werden in 1l VE-Wasser gelöst. Nun wird unter Rühren die Kalilauge-/Ammoniaklösung in zunächst 90% der Silberlösung getropft. Nachdem sich der anfänglich auftretende braune Niederschlag wieder gelöst hat, wird auch der Rest der Silberlösung zugegeben.
   Zu der klaren farblosen Lösung werden noch 2ml Jodtinktur zugesetzt (5g Jod in 95g Ethanol).

### Aktivierung:

2g Aluminiumoxidplättchen mit einer Schichtdicke von 350 bis 400 nm werden in 50ml Aktivierungslösung suspendiert und 10min bei Raumtemperatur behandelt. Die Aktivierungslösung enthält 0,1 ml Aktivierungskonzentrat in 1l VE-Wasser. Danach wird das Material abgesaugt und mit VE-Wasser gewaschen.

### Beschichtung:

Zur Beschichtung werden die aktivierten Aluminiumoxidplättchen in 100ml VE-Wasser aufgeschlemmt und mit 10ml Reduktionskonzentrat versetzt. Diese Suspension wird unter Rühren in die Silberlösung gegeben und 30min bei Raumtemperatur gerührt.
Danach filtriert man das Material ab, wäscht mit VE-Wasser und Ethanol nach und trocknet 12h bei 110°C. Das so hergestellte Pigment weist neben attraktivem Silberglanz und Deckvermögen einen ausgeprägten Kristalleffekt auf.

### Beispiel 2:

Aufbringung von Metallschichten durch Vakuumbedampfung

In einer Hochvakuumverdampfungsanlage mit Magnetronkathode werden 200g Aluminiumoxidplättchen mit einer Schichtdicke von 300 bis 350 nm in die Substratvorrichtung gebracht. Nach dem Abpumpen der Beschichtungskammer auf 10⁻⁵ Torr lässt man Argon bis zu einem Druck von 10⁻³ Torr einströmen. Zunächst wird für 10min die Oberfläche des Aluminium-Targets durch Ionenbeschuß gereinigt, wobei eine Blende das Pulver abdeckt.
Anschließend findet unter ständiger Bewegung des Pulvers bei herausgeschwenkter Blende die Beschichtung mit Aluminium bei einem Arbeitsdruck von 10⁻³ Torr für die Dauer von ca. 150min statt, wobei die Dauer der Beschichtung in Abhängigkeit von der gewünschten Schichtdicke eingestellt wird.
Das so hergestellte Pigment weist neben hoher Reflektivität und hohem Deckvermögen einen ausgeprägten Kristalleffekt auf.

## Patentansprüche

1. Pigment mit Metallglanz, umfassend ein plättchenförmiges Substrat mit einer Dicke zwischen 300 und 500 nm, welches von einer Metallschicht vollständig umhüllt ist, wobei das Substrat aus Einkristallen von Aluminiumoxid mit einem Anteil an Titanoxid besteht:

2. Pigment gemäß Anspruch 1, bei dem der Anteil an Titanoxid 0,1- 4 Gew. -% beträgt

3. Pigment gemäß einem der Ansprüche 1 oder 2, welches ein Substrat mit einem mittleren Teilchendurchmesser von 5 bis 60 µm und einem Verhältnis des mittleren Teilchendurchmessers zur mittleren Teilchendicke von größer als 20 aufweist.

4. Pigment gemäß einem oder mehreren der Ansprüche 1 bis 3, wobei die Metallschicht aus Aluminium, Titan, Chrom, Nickel, Silber, Zink, Molybdän, Tantal, Wolfram, Palladium, Kupfer, Gold, Platin oder diese enthaltenden Legierungen besteht.

5. Pigment gemäß einem oder mehreren der Ansprüche 1 bis 4, wobei die Dicke der Metallschicht 15 bis 100 nm beträgt

6. Verfahren zur Herstellung des Pigmentes gemäß Anspruch 1, indem das Substrat in einem wässrigen und/oder Lösemittel enthaltenden Medium in Anwesenheit einer Metallverbindung suspendiert und nach Zugabe eines Reduktionsmittels die Metallschicht auf dem Substrat abgeschieden wird.

7. Verfahren zur Herstellung des Pigmentes gemäß Anspruch 1, indem das in einem Wirbelbett fluidisierte Substrat mit Metallen beschichtet wird, die durch Gasphasenzersetzung der entsprechenden flüchtigen Metallverbindungen erhalten werden.

8. Verfahren zur Herstellung des Pigmentes gemäß Anspruch 1, indem im Hochvakuum durch Sputtern oder thermisches Verdampfen die entsprechenden Metalle auf dem Substrat abgeschieden werden, wobei das Substrat während des Beschichtungsvorgangs gleichmäßig in Bewegung gehalten wird.

9. Verwendung des Pigmentes gemäß einem oder mehreren der Ansprüche 1 bis 5 in Farben, Lacken, Druckfarben, Kunststoffen, kosmetischen Formulierungen, keramischen Materialien, Gläsern, Papier, zur Lasermarkierung, in Sicherheitsanwendungen sowie in Trockenpräparaten und Pigmentpräparationen.

10. Farben, Lacken, Druckfarben, Kunststoffe, kosmetische Formulierungen, keramische Materialien, Gläser, Papier, Trockenpräparate, Pigmentpräparationen und Materialien für Sicherheitsanwendungen, enthaltend ein Pigment gemäß einem oder mehreren der Ansprüche 1 bis 5.

## Claims

1. Pigment having metallic lustre, comprising a flake-form substrate having a thickness between 300 and 500 nm which is completely surrounded by a metal layer, where the substrate consists of aluminium oxide single crystals having a content of titanium oxide.

2. Pigment according to Claim 1, in which the proportion of titanium oxide is 0.1-4% by weight.

3. Pigment according to one of Claims 1 or 2, which has a substrate having an average particle diameter of 5 to 60 µm and a ratio between the average particle diameter and the average particle thickness of greater than 20.

4. Pigment according to one or more of Claims 1 to 3, where the metal layer consists of aluminium, titanium, chromium, nickel, silver, zinc, molybdenum, tantalum, tungsten, palladium, copper, gold, platinum or alloys comprising these.

5. Pigment according to one or more of Claims 1 to 4, where the thickness of the metal layer is 15 to 100 nm.

6. Process for the preparation of the pigment according to Claim 1, in which the substrate is suspended in an aqueous and/or solvent-containing medium in the presence of a metal compound and, after addition of a reducing agent, the metal layer is deposited on the substrate.

7. Process for the preparation of the pigment according to Claim 1, in which the substrate fluidised in a fluidised bed is coated with metals obtained by gas-phase decomposition of the corresponding volatile metal compounds.

8. Process for the preparation of the pigment according to Claim 1, in which the corresponding metals are deposited on the substrate in a high vacuum by sputtering or thermal evaporation, with the substrate being kept uniformly in motion during the coating operation.

9. Use of the pigment according to one or more of Claims 1 to 5 in paints, surface coatings, printing inks, plastics, cosmetic formulations, ceramic materials, glasses, paper, for laser marking, in security applications and in dry preparations and pigment compositions.

10. Paints, surface coatings, printing inks, plastics, cosmetic formulations, ceramic materials, glasses, paper, dry preparations, pigment compositions and materials for security applications comprising a pigment according to one or more of Claims 1 to 5.

## Revendications

1. Pigment présentant un lustre métallique, comprenant un substrat sous forme de flocons présentant une épaisseur entre 300 et 500 nm, lequel substrat est complètement entouré par une couche métallique, dans lequel le substrat est constitué par des monocristaux d'oxyde d'aluminium présentant une teneur en oxyde de titane.

2. Pigment selon la revendication 1, dans lequel la proportion d'oxyde de titane est de 0,1-4% en poids.

3. Pigment selon une des revendications 1 ou 2, lequel comporte un substrat présentant un diamètre de particule moyen de 5 à 60 µm et un rapport entre le diamètre de particule moyen et l'épaisseur de particule moyenne supérieur à 20.

4. Pigment selon une ou plusieurs des revendications 1 à 3, dans lequel la couche métallique est constituée par aluminium, titane, chrome, nickel, argent, zinc, molybdène, tantale, tungstène, palladium, cuivre, or, platine ou des alliages les comprenant.

5. Pigment selon une ou plusieurs des revendications 1 à 4, dans lequel l'épaisseur de la couche métallique est de 15 à 100 nm.

6. Procédé pour la préparation du pigment selon la revendication 1, dans lequel le substrat est suspendu dans un milieu aqueux et/ou contenant un solvant en présence d'un composé métallique et, après addition d'un agent réducteur, la couche métallique est déposée sur le substrat.

7. Procédé pour la préparation du pigment selon la revendication 1, dans lequel le substrat fluidisé dans un lit fluidisé est revêtu de métaux obtenus par décomposition en phase gazeuse des composés métalliques volatiles correspondants.

8. Procédé pour la préparation du pigment selon la revendication 1, dans lequel les métaux correspondants sont déposées sur le substrat sous un vide poussé par pulvérisation ou évaporation thermique, le substrat étant maintenu de façon uniforme en déplacement pendant l'opération de revêtement.

9. Utilisation du pigment selon une ou plusieurs des revendications 1 à 5 dans des peintures, des revêtements de surface, des encres d'impression, des matières plastiques, des formulations cosmétiques, des matériaux de céramique, des verres, du papier, pour un marquage laser, dans des applications de sécurité et dans des préparations sèches et des compositions de pigments.

10. Peintures, revêtements de surface, encres d'impression, matières plastiques, formulations cosmétiques, matériaux de céramique, verres, papier, préparations sèches, compositions de pigments et matériaux pour des applications de sécurité comprenant un pigment selon une ou plusieurs des revendications 1 à 5.
